# EUROPEAN PATENT APPLICATION

(11) **EP 2 154 267 A2**
(43) Date of publication of application: **17.02.2010**
(21) Application number: 09251907.3
(22) Date of filing: 30.07.2009
(51) Int. Cl.: C23F 11/08, G01N 21/33

(54) **Apparatus for in-line testing of a chemical in a water containing system**

(30) Priority: 14.08.2008 GB 0814873; 25.03.2009 GB 0905056
(71) Applicant: Sentinel Performance Solutions Ltd, Runcorn Cheshire WA7 4QX (GB)
(72) Inventor: Marshall, Alan, Warrington Cheshire WA4 5HG (GB); Mawer, John Henry, Great Boughton Chester CH3 5XH (GB); Day, Paul, 2000 Antwerpen (BE)
(74) Representative: Fenwick, Elizabeth Anne

(57) **Abstract**

An apparatus, suitable for in-line testing of the concentration of a selected chemical, and thus the concentration of any preparation in which it is contained in a water containing system, comprises:
• a UV- light transparent portion (10) through which water will pass in normal operation of the system,
• at least one UV light source (8,9) selected to emit UV light at a predetermined wavelength known to be absorbed by the selected chemical,
• at least one photo cell (11),
• a pre-calibrated measuring and visual display means,
• a power source (7),
• means to actuate the power source,
arranged so that in order to initiate testing the power source (7) is activated, UV light passes from the UV light source (8,9) through water in the UV light-transparent portion (10) and is received by the photo cell (11), which measures how much UV light has been absorbed by the chemical in the water at the predetermined wavelength and this in turn indicates whether the concentration of the chemical is acceptable and provides a visual display thereof.

## Description

The invention relates to a method and apparatus suitable for in-line monitoring of the concentration of a chemical in a water-containing system e.g. a central heating system.

Any system constructed of metal and containing water will, to some degree, suffer from the effects of corrosion and hardness scale formation depending on the quality of the water used in that system and the temperature to which the water is subjected. Systems will normally fall into two groups, open systems such as systems using cooling towers or closed systems such as chilled water systems or heating systems. The effects of corrosion are seen through system failure and the build up of corrosion debris whereas the effects of water hardness scale formation are a reduction in system performance, a build up of sludge and again system failure. To minimise the effects of corrosion and hardness scale formation, chemicals usually termed 'inhibitors', are added to these systems at an appropriate concentration specific to that chemical. The introduction of the appropriate level of inhibitor and maintenance of that level are paramount in maintaining the systems as free as possible from corrosion and hardness scale formation. To maintain correct levels of inhibitor requires regular monitoring of inhibitor concentration by testing and then subsequent addition of chemical where levels are found to be low.

Known systems for testing of levels of inhibitors require the removal of a sample of water from the system and are normally carried out by manual testing for a component contained in the inhibitor - so-called 'wet testing'. Tests are often based on colorimetric analysis, titration or use of a chemical sensitive test strip. Each of these methods has drawbacks:
- Varying amounts of time are required to carry out the test which discourages engineers from testing
- They have poor levels of accuracy which can result in over treating or under treating with inhibitor
- Very often use hazardous chemicals
- Involve the need to take samples from hot systems resulting in a risk from scalding or damage to the system itself

The concept of the present invention is particularly applicable to the use of UV light and is described mainly in that context, but can be extended to the use of light having wavelength in the visible or infrared wavelength ranges.

According to one aspect of the present invention the apparatus, suitable for in-line testing of the concentration of a selected chemical, and thus the concentration of any preparation in which it is contained in a water containing system, comprises :
- a light transparent portion through which water will pass in normal operation of the system,
- at least one light source selected to emit light at a predetermined wavelength known to be absorbed by the selected chemical,
- at least one photo cell,
- a pre-calibrated measuring and visual display means,
- a power source
- means to actuate the power source,
arranged so that in order to initiate testing the power source is activated, light passes from the light source through water in the light-transparent portion and is received by the photo cell, which measures how much light has been absorbed by the chemical in the water at the predetermined wavelength and this in turn indicates whether the concentration of the chemical is acceptable and provides a visual display thereof.

According to a preferred aspect of the present invention the apparatus, suitable for in-line testing of the concentration of a selected chemical, and thus the concentration of any preparation in which it is contained in a water containing system, comprises :
- a UV- light transparent portion through which water will pass in normal operation of the system,
- at least one UV light source selected to emit UV light at a predetermined wavelength known to be absorbed by the selected chemical,
- at least one photo cell,
- a pre-calibrated measuring and visual display means,
- a power source
- means to actuate the power source,
arranged so that in order to initiate testing the power source is activated, UV light passes from the UV light source through water in the UV light-transparent portion and is received by the photo cell, which measures how much UV light has been absorbed by the chemical in the water at the predetermined wavelength and this in turn indicates whether the concentration of the chemical is acceptable and provides a visual display thereof.

According to yet another aspect of the present invention, a method of in-line testing of the concentration of a selected chemical in water comprises passing light at a predetermined wavelength from a light source through the water to a photo cell which detects how much light has been absorbed by the selected chemical, the resulting signal is then transmitted to a pre-calibrated measuring and display means which calculates the concentration of the selected chemical and by reference to predetermined concentration thresholds provides a visual display of acceptable or non-acceptable additive concentration.

According to another preferred aspect of the present invention, a method of in-line testing of the concentration of a selected chemical in water comprises passing UV light at a predetermined wavelength from a UV light source through the water to a photo cell which detects how much UV light has been absorbed by the selected chemical, the resulting signal is then transmitted to a pre-calibrated measuring and display means which calculates the concentration of the selected chemical and by reference to predetermined concentration thresholds provides a visual display of acceptable or non-acceptable additive concentration.

The method and apparatus can be used in the initial commissioning (set-up) of a water containing system and/or as ongoing monitoring of that system. The objective of this invention is to remove the hazards, reduce the amount of time required and improve the accuracy of the test.

An inhibitor added to a central heating system would include various chemical compounds e.g. A+B+C=D. These compounds would have functions such as, but not limited, to being active in controlling scale or corrosion, or adjuncts thereto, or performing other functions in the formulation such as maintaining its stability or preserving it from microbiological attack, or specifically added to the formulation for the purpose of detection by light absorbance.

In the first example a compound commonly found in corrosion control preparations was selected, namely 1,2,3 Benzotriazole and tests were performed to establish the UV light wavelength at which it absorbed UV light. Having established that peak absorbance occurred at 290 nm, a UV light source (e.g. an LED) emitting at that same wavelength was selected. Experimentation established an excellent linear correlation between UV light absorption levels and concentration levels of the selected compound. Use of a second UV light source emitting at a different wavelength was used to exclude absorption from the background colour (typically derived from soluble and insoluble metal corrosion products) of the water in the system. Using suitable electronic circuitry, the resulting absorption could be translated into a visual display of 'traffic light' type warning lights indicating whether the composition (e.g. an inhibitor) was present in sufficient concentrations to be efficacious.

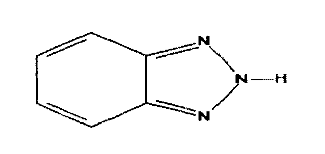

In this example, tests were undertaken using 10mm cuvettes with various concentrations of the inhibitor in tap water at the 290nm wavelength.

his gave absorbencies up to 1.8 (attenuation factor of 0.015) for a 1% solution with a close to linear relationship to concentration.

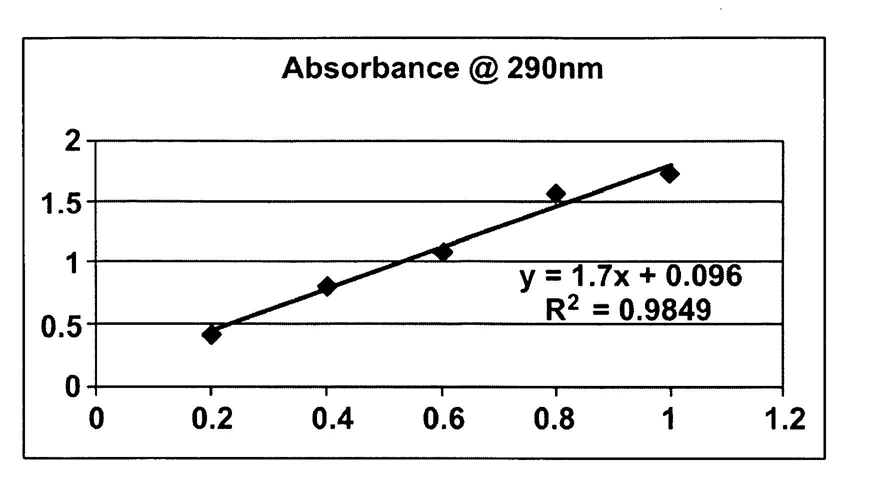

This, as an example, provides a basis for the detection system.

As a second example, an inhibitor preparation contained a compound added specifically for the purpose of detection by light absorbance. The compound selected was 2,2'-[(1,1'-Biphenyl)-4,4'-diyldi-2,1-ethenediyl]-bis-benzenesulfonic acid disodium salt.
Tests were performed to establish the UV light wavelength at which it absorbed UV light. Having established that peak absorbance occurred at 350 nm, a UV light source (e.g. an LED) emitting at that same wavelength was selected. Experimentation established an excellent linear correlation between UV light absorption levels and concentration levels of the selected compound.

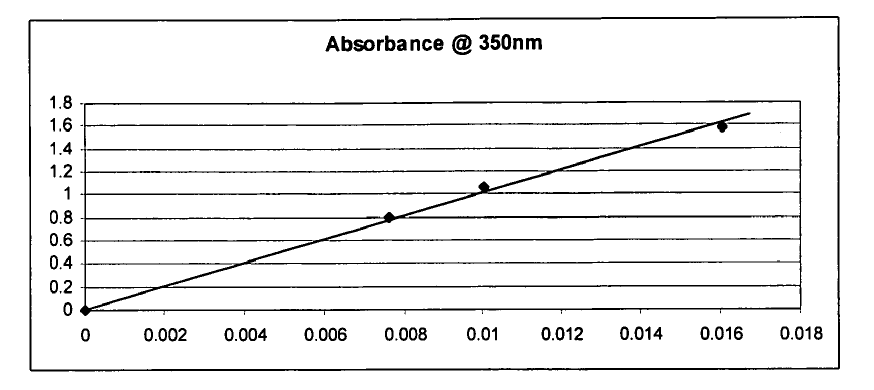

This, as a second example, provides a basis for the detection system

Thus the principle was established that compounds in a formulation integral to the normal function of a central heating additive or specifically added to the formulation for the purpose of detection by light absorbance may be utilised for detection by light absorbance and calculation of concentration of the additive.

This invention is particularly applicable to the monitoring of concentration of a component of an inhibitor in a domestic central heating system, although it could be equally effective in other water-containing systems and for additives other than inhibitors. A domestic central heating system consists principally of a boiler to heat water, a pump to circulate heated water around the system and radiators to transmit heat into the living/working space. Metals used in systems would normally be mild steel, copper, brass and aluminium. The heat and metals involved provide an ideal environment for water hardness scale formation and metal corrosion with the attendant problems previously described.

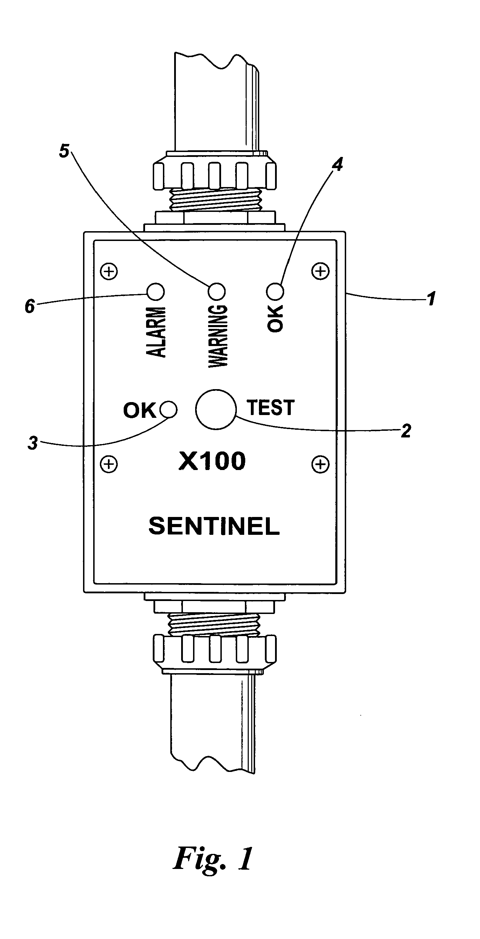

The power source would normally be a battery and preferably the means to actuate the power source would be a push button ('Test' in Figure 1). The visual display should be simple to read and interpret and in a preferred embodiment consists of a red/amber/green light (e.g. LEDs) indicating fail/warn/pass.

Preferably the apparatus will be contained in a housing for safety and convenience. For example, the housing may be of metal or plastic.

For safety reasons, preferably the apparatus will contain no user-serviceable parts and will have the following indicators and controls:
A push button Test Switch, a Power Indicator (See Figure 1) and Results LEDs.

The Results are interpreted as follows
■ Red LED - Inhibitor Level Very Low ('Alarm' in XFigure 1) - top up.
■ Amber LED - Inhibitor Present but below recommendations ('Warning' in Figure 1) - top up.
■ Green LED - Inhibitor Present and within recommendations ('OK' in Figure 1).

When a reading is required the operator depresses and holds the "Test" button for a few seconds. The electronics are powered and a measurement cycle is initiated. The results are displayed through the results LEDs.

The aims of the present invention are to provide an instant indication of system treatment levels. This should give the comfort factor of knowing the system is treated correctly and correct treatment improves system efficiency and longevity of system components.

### Extension of the Idea

The concept of the unit has been extended to encapsulate not only the monitoring of the level of inhibitor but to develop the capabilities of the unit so that it can be used to commission central heating systems.

One of the doubts when a central heating system is commissioned is whether the chemical supplier's instructions have been followed correctly and that the procedure has been correctly carried out. The boiler manufacturers believe that if the commissioning process is carried out correctly it will reduce the number of costly call-backs to rectify faults with the water system of the central heating system.

The extension of the original idea is to produce a unit that can monitor and control the commissioning process and provide proof or otherwise that the process has been carried out correctly.

### The Commissioning Process

The new central heating system is filled with water and a cleaner is added to give the correct concentration.

The fluid in the system is then circulated for 1 hour to clean the system of metal oxides, solder and flux residues and to remove any material that entered the system during the fabrication process.

After the 1 hour circulation period the system is flushed with water to remove all the suspended and soluble material in the water. This flushing process also removes all traces of the cleaner.

Once the system has been flushed the system water is dosed with the correct concentration of inhibitor.

### Extended Product Concept

■ A unit fitted to the system to give a visual display of the commissioning process
■ Permanent record of the successful completion of the commissioning process
■ Display of a unique number when the commissioning has been carried out correctly and this number can be texted to a database to confirm the commissioning has been carried out correctly
■ On-line display of inhibitor levels triggered by push button
■ Simple to understand red / green light (pass/fail)
■ Indication to top-up inhibitor

The proposed instrument will comprise a sensor body tapped each end with 3/4"BSP threads for inclusion directly into the central heating circuit.
■ This item will contain no user serviceable parts and have the following indicators and controls.
■ Red LED and Green LED for the cleaning process
■ Red LED and Green LED for the flushing process
■ Red LED and Green LED for the inhibiting process (introduction of inhibitor)
■ Push Button Test Switch and Power Indicator
■ Results LEDs for subsequent and ongoing testing of the inhibitor
■ The Results are interpreted as follows
■ Red LED - Inhibitor Level Very Low
■ Amber LED - Inhibitor Present but below recommendations
■ Green LED - Inhibitor Present and within recommendations

The cleaning chemical and inhibitor will be introduced into the system at any convenient location. Conventional liquids or gels can be used to dose the system but for convenience it is envisaged that the cleaning chemical and the inhibitor will be introduced into the system using the Sentinel Rapid Dose concept. Sentinel Rapid-Dose is a recyclable, pressurised canister pre-filled with inhibitor (or cleaner) that can be used to dose any indirect central heating system.

Preferably the apparatus is provided with connection means to facilitate in-line connection into the water containing system, e.g. a unit tapped at each end with 3/4"BSP threads for inclusion directly into a central heating circuit.

The concept relies on the absorbance of light of wavelength in the UV, visible or IR ranges by one or more components of cleaning or inhibitor treatment formulations. In the case of UV light, the source is preferably a light emitting diode (LED) which is preselected to emit UV light at a wavelength which will be absorbed by the chemical selected to be monitored in the water. In the simplest case, different additives used sequentially in the commissioning protocol may be detected at the same wavelength by a single LED so long as the different additives contain the same detected component, or components that absorb at the same pre-selected wavelength, By extension, it is feasible that the concept could include more than one LED emitting at different wavelengths to detect different chemicals in respective additives.

The apparatus preferably also has automatic compensation for background colouration of the water (typically derived from soluble and insoluble metal corrosion products) by the inclusion of a second UV light source set at a different wavelength to the first UV light source. The second UV light emitter/diode will look at a wide band of wavelengths and anything found that is different to the wavelength of the first UV light source is subtracted from the reading.

The UV light-transparent portion, through which water will pass in normal operation of the system, should be of a material able to withstand chemical attack from the water and should be stable at the operating temperature of the water containing system, which in the case of a central heating system would often be in the range 60 to 80 degrees Celsius. Glass would satisfy these requirements but is by nature fragile. A clear plastic would be preferable if a formulation with the ability to withstand the operating conditions could be found.

It will be appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable combination.

Embodiments of the present invention will now be described with reference to the accompanying drawings in which;
Figure 1 is a front view of an apparatus for inline monitoring according to one embodiment of the present invention.
Figure 2 are schematic views of the components of the apparatus shown in Figure 1.
Figure 3 is a rear perspective view of the shown in Figures 1 and 2.
Figure 4 is a front view of an apparatus for inline testing during commissioning and ongoing monitoring according to a second embodiment of the present invention.
Figure 5 is a chart showing the monitoring and controlling of the commissioning process using the apparatus in Figure 4.

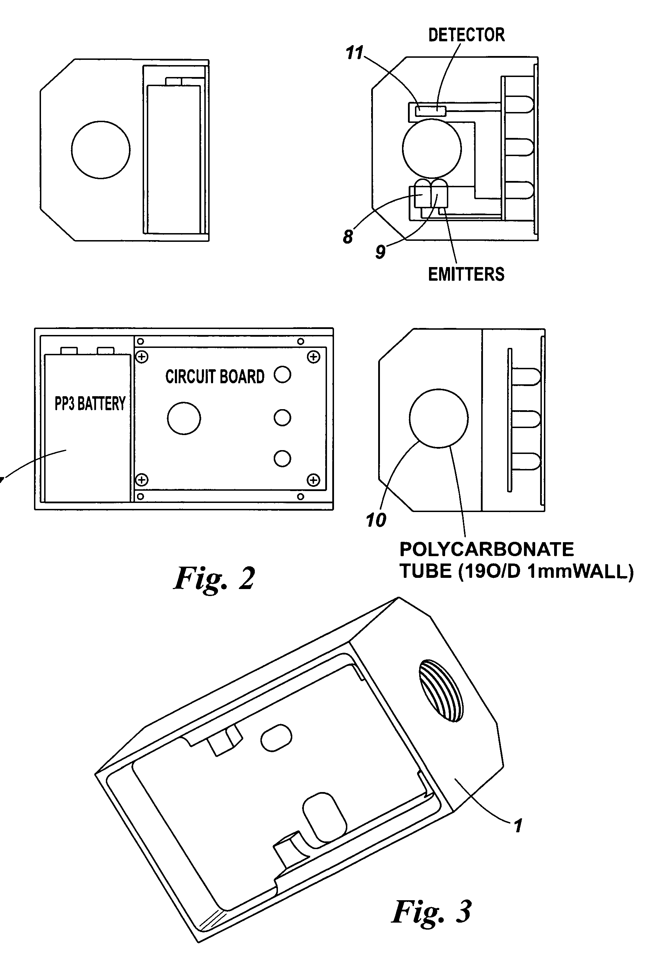

Referring to Figures 1 and 3, the apparatus is shown fitted in-line to the pipe work of a central heating system containing the inhibitor by means of 3/4"BSP threads at each end. The casing **1** which is of injection-moulded plastic (see Figure 3) is provided with a "Test' button **2,** an 'On' LED indicator **3** and a visual display of three LED indicators **4,5,6** marked 'OK'(green), 'Warning'(amber) and 'Alarm'(red) respectively.

Referring to Figure 2, the power source is a PP3 battery **7** and there are two emitters **8,9** (for example UV light sources) which are LEDs. The first emitter **8** is set to emit for example UV light at a wavelength known to be absorbed by the chemical in the water selected to be tested (for example 290nm for 1,2,3, Benzotriazole), and the second emitter **9** is set to emit for example UV light at a different wavelength or range of wavelengths away from the wavelength of the first emitter. The UV light -transparent portion through which water passes is a tube **10** made of a material able to withstand chemical attack from the water and stable at the operating temperature of the water-containing system with a wall thickness of approximately 1 mm. The detector **11** is a photo cell.

In use, as an example, an operative pushes the test button **2,** causing power to flow to the two UV light emitters **8,9** (LEDs). UV light passes though the water in the tube **10** and to the photo cell **11.** The level of absorption resulting from the selected chemical is calculated by reference to pre-determined calibration equation and this provides the concentration of that chemical. If the concentration falls within a predetermined range of acceptability the green ('OK') LED **4** is illuminated, if it is within a predetermined range below that the amber ('Warning') LED **5** is illuminated and if it is below that level the red ('Alarm') LED **6** is illuminated. The operative can see immediately whether additional inhibitor needs to be added.

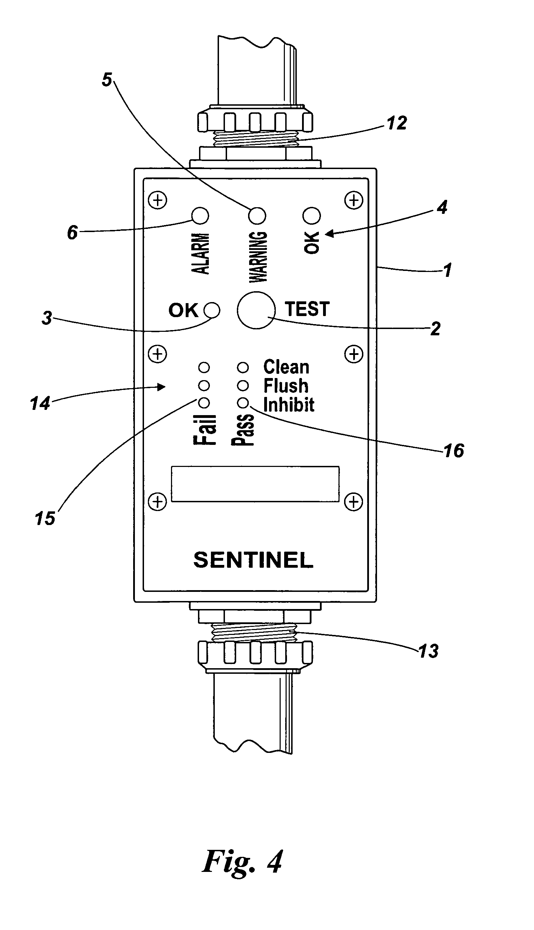

Referring to Figure 4, the apparatus is shown fitted in-line to the pipe work of a central heating system containing the inhibitor by means of 3/4"BSP threads **12,13** at each end. The casing **1** which is of injection-moulded plastic (see Figure 3) is provided with a "Test' button **2,** an 'On' LED indicator **3** and a visual display of three LED indicators **4,5,6** marked 'OK'(green), 'Warning'(amber) and 'Alarm'(red) respectively. There is also a display **14** of LEDs consisting of a row of three red 'fail' LEDs **15** and a row of three green 'Pass' LEDs **16.** The top two LEDs relate to the cleaning phase, the middle two to the flushing phase and the bottom two to the inhibitor phase.

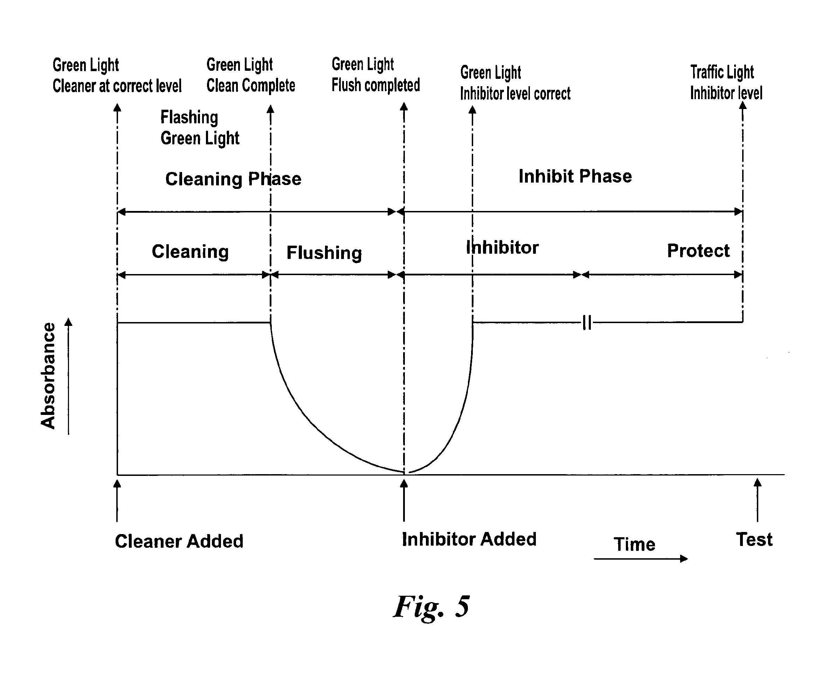

Referring to Figures 4 and 5, initially when the unit is switched on, the Red 'fail' LED lights **15** for clean, flush and inhibit will be lit to indicate a 'fail' situation. When the cleaner is introduced into the system at the right concentration as detected by the detector, the top Green LED light for the clean in row **16** will be lit and the top Red LED in row **15** will be turned off.

Once the top Green LED light for the clean is lit it will activate a timer which will run for 1 hour. During this period the top Green LED light for the clean will flash to indicate the cleaning process is being carried out. When the 1 hour period has elapsed the Green LED light for the clean will remain on constantly to indicate the clean has finished and has been carried out correctly. This indicates a 'pass' condition.

The next stage of the commissioning sequence is to flush the system. When the system has been flushed and the absorbance as detected by the detector has been reduced to zero, the middle Green LED light for the flush in row **16** will be lit and the middle Red LED in row **15** will be turned off. The middle Green LED light for the flush will remain on constantly to indicate the flush has finished and has been carried out correctly. This indicates a 'pass' condition.

The next stage of the commissioning sequence is to inhibit the system. When the inhibitor has been introduced into the system and the absorbance as detected by the detector is at the correct level for the right level of inhibitor the bottom Green LED light for the inhibitor in row **16** will be lit and the bottom Red LED in row **15** will be turned off. The Green LED light for the inhibitor will remain on constantly to indicate the inhibitor has been introduced to the correct concentration. This indicates a 'pass' condition.

When the three Green LED lights in row **16** are lit indicating a 'pass' condition for clean, flush and inhibition the processor will generate a unique number which will be displayed on the LCD screen. This unique number can be texted via a mobile phone to database to confirm the commissioning process has been carried out correctly.

Once the unique number has been displayed the three Green LED lights in row **16** will be turned off.

The level of inhibitor in the system can subsequently be determined by pressing the test button.
■ Results LEDs for subsequent and ongoing testing of the inhibitor
■ The Results are interpreted as follows
■ Red LED **6**- Inhibitor Level Very Low
■ Amber LED **5**- Inhibitor Present but below recommendations
■ Green LED **4**- Inhibitor Present and within recommendations

At the same time the test button is pressed the appropriate 'pass' and 'fail' Green or Red LEDs **15,16** will be lit showing the history of the commissioning process.

### Detection of Cleaner and Inhibitor

The concept relies on the absorbance of light of wavelength in the UV, visible or IR ranges by one or more components of cleaning or inhibitor treatment formulations. In the case of UV light, the source is preferably a light emitting diode (LED) which is preselected to emit UV light at a wavelength which will be absorbed by the chemical selected to be monitored in the water.

In the simplest case, different additives used sequentially in the commissioning protocol may be detected at the same wavelength by a single LED so long as the different chemicals contain the same detected component, or components that absorb at the same pre-selected wavelength, By extension, it is feasible that the concept could include more than one LED emitting at different wavelengths to detect different chemicals in respective additives.

### Generation of a Unique Number

A sequential serial number will be programmed into each device at manufacture.

This number will be read by the processor program and converted to an alpha-numeric sequence using base 36 arithmetic (0-9 + A-Z) giving a range of 0-60 million combinations with 5 characters.

The system status will be made printable and appended to the serial number. A 16 bit CRC (cyclic redundancy check) is calculated and also appended giving an 8 character sequence which is displayed on the LCD.

A CRC is not something that can be easily calculated and will give a good degree of 'tamper proof whilst being simple to implement.

A code in the processor will check to decipher the serial number and check the CRC to see that is genuine.

### Reset Facility

The unit will have a facility to reset the 'pass' and 'fail' lights such that the Red LED lights for clean, flush and inhibit will be lit to indicate a 'fail' situation. This is to allow the unit to monitor and control any subsequent cleans that may be carried out during the life-time of the central heating system.

## Claims

1. An apparatus, suitable for in-line testing of the concentration of a selected chemical, and thus the concentration of any preparation in which it is contained in a water containing system, comprises:
• a light transparent portion through which water will pass in normal operation of the system,
• at least one light source selected to emit light at a predetermined wavelength known to be absorbed by the selected chemical,
• at least one photo cell,
• a pre-calibrated measuring and visual display means,
• a power source
• means to actuate the power source,
arranged so that in order to initiate testing the power source is activated, light passes from the light source through water in the light-transparent portion and is received by the photo cell, which measures how much light has been absorbed by the chemical in the water at the predetermined wavelength and this in turn indicates whether the concentration of the chemical is acceptable and provides a visual display thereof.

2. An apparatus, suitable for in-line testing of the concentration of a selected chemical, and thus the concentration of any preparation in which it is contained in a water containing system, comprises :
• a UV- light transparent portion through which water will pass in normal operation of the system,
• at least one UV light source selected to emit UV light at a predetermined wavelength known to be absorbed by the selected chemical,
• at least one photo cell,
• a pre-calibrated measuring and visual display means,
• a power source
• means to actuate the power source,
arranged so that in order to initiate testing the power source is activated, UV light passes from the UV light source through water in the UV light-transparent portion and is received by the photo cell, which measures how much UV light has been absorbed by the chemical in the water at the predetermined wavelength and this in turn indicates whether the concentration of the chemical is acceptable and provides a visual display thereof.

3. A method of in-line testing of the concentration of a selected chemical in water comprises passing light at a predetermined wavelength from a light source through the water to a photo cell which detects how much light has been absorbed by the selected chemical, the resulting signal is then transmitted to a pre-calibrated measuring and display means which calculates the concentration of the selected chemical and by reference to predetermined concentration thresholds provides a visual display of acceptable or non-acceptable additive concentration.

4. A method of in-line testing of the concentration of a selected chemical in water comprises passing UV light at a predetermined wavelength from a UV light source through the water to a photo cell which detects how much UV light has been absorbed by the selected chemical, the resulting signal is then transmitted to a pre-calibrated measuring and display means which calculates the concentration of the selected chemical and by reference to predetermined concentration thresholds provides a visual display of acceptable or non-acceptable additive concentration.

5. An apparatus or method according to any preceding claim which is used in the initial commissioning (set-up) of a water-containing system.

6. An apparatus or method according to any preceding claim which is used in ongoing monitoring of a water-containing system.

7. An apparatus or method according to any preceding claim which is used to monitor an inhibitor.

8. An apparatus or method according to any preceding claim in which the water-containing system is a central heating system.

9. An apparatus or method according to any preceding claim in which the selected chemical in the water has been specifically added.

10. An apparatus or method according to any of claims 1 to 8 in which the selected chemical in the water has been not been specifically added, but is usually present in the formulation.

11. An apparatus or method according to any preceding claim in which the light source comprises al least one light emitting diode (LED).

12. An apparatus or method according to any preceding claim in which the visual display comprises at least one light emitting diode (LED).

13. An apparatus or method according to any preceding claim in which the visual display comprises traffic light warning lights of red, amber and green.

14. An apparatus or method according to any preceding claim in which the means to actuate the power source comprise a test button.

15. An apparatus or method according to any preceding claim which includes a housing to contain the component parts.
